# EUROPEAN PATENT SPECIFICATION

(11) **EP 4 615 818 B1**
(45) Date of publication and mention of the grant of the patent: **11.02.2026**
(21) Application number: 24802247.7
(22) Date of filing: 11.11.2024
(51) Int. Cl.: C07C 67/08, C07C 69/84

(54) **PROCESS FOR THE PREPARATION OF 2-(METH)ACRYLOYLOXY BENZOIC ACID ESTERS USING A CATALYST SYSTEM**
VERFAHREN ZUR HERSTELLUNG VON 2-(METH)ACRYLOYLOXYBENZOESÄUREESTERN UNTER VERWENDUNG EINES KATALYSATORSYSTEMS
PROCÉDÉ DE PRÉPARATION D'ESTERS D'ACIDE 2-(MÉTH)ACRYLOYLOXY BENZOÏQUE À L'AIDE D'UN SYSTÈME CATALYTIQUE

(30) Priority: 20.11.2023 EP 23210851; 27.11.2023 EP 23212325
(43) Date of publication of application: 17.09.2025
(73) Proprietor: Evonik Operations GmbH, 45128 Essen (DE)
(72) Inventor: WALTER, Kristine, 64354 Reinheim (DE); SAAL, Doris, 64625 Bensheim (DE); BESTGEN, Sebastian, 97080 Würzburg (DE); SCHÜTZ, Thorben, 64665 Alsbach-Hähnlein (DE)
(74) Representative: Evonik Patent Association
(86) International application number: PCT/EP2024/081842
(87) International publication number: WO 2025/108750

(56) References cited:
- WO-A1-2019/213588
- JP-A- 2022 147 037

## Description

The present invention is directed to a solvent-free process for the preparation of 2-(meth)acryloyloxy benzoic acid esters wherein 2-hydroxybenzoates are reacted with (meth)acrylic acid anhydride in the presence of a mixture of an earth alkaline salt and an alkaline salt as catalyst.

These polymerizable monomers can be used in a variety of applications, including the use as reactive diluent in photopolymerization processes, such as lithography-based photopolymerization processes, for example high temperature lithography-based processes conducted at temperatures greater than 90°C. The monomer allows for good processibility in additive manufacturing techniques and may yield cured products having thermomechanical properties that are desirable for various applications, including for the formation of medical devices and/or those items used in an intraoral environment, e.g., intraoral devices, such as aligners, expanders or spacers.

### State of the art

WO 2019/213588 is directed to photopolymerizable monomers and discloses the preparation of certain 2-(meth)acryloyloxy benzoic acid esters by reacting 2-hydroxy benzoates with (meth)acryloyl chloride.

The use of (meth)acrylic anhydride as reagent is described for the substrate menthyl salicylate in the presence of DMAP as catalyst without the use of a solvent (see Example 3, [0185]).

A solvent-free preparation of 2-(meth)acryloyloxy benzoic acid esters in the presence of a mixture of an earth alkaline salt and an alkaline salt as catalyst is not described.

Liska et. al. (Journal of Polymer Science 2021 (Hoboken, NJ, United States), Volume 59, Issue 19, Pages 2154-2169) relates to a method for producing salicylate methacrylates in dichloromethane using stochiometric amounts of triethyl amine and methacryloyl chloride. The precipitated ammonium chloride was removed by filtration. The filtrate was washed, and the organic layer was dried over Na₂SO₄, filtered and stabilized, Subsequently, the solvent was evaporated. The crude product was purified by silica chromatography.

A solvent-free preparation of 2-(meth)acryloyloxy benzoic acid esters in the presence of a mixture of an earth alkaline salt and an alkaline salt as catalyst is not described.

WO 2019/224193 relates to a method for preparing keto-functionalized aromatic (meth)acrylates by reacting keto-functionalized aromatic alcohols or keto-functionalized aromatic amines with (meth)acrylic anhydride having a content of (meth)acrylic acid anhydride of less than 4.5%. Exemplary products are benzophenone derivatives, prepared in the presence of catalytic amounts of concentrated sulfuric acid or aqueous sodium hydroxide solution.

The solvent-free preparation of 2-(meth)acryloyloxy benzoic acid esters in the presence of a mixture of an earth alkaline salt and an alkaline salt as catalyst is not described.

WO 2020/035315 relates to a method for preparing (meth)acrylic acid esters of primary, secondary and tertiary alcohols as well as phenols using (meth)acrylic anhydride in the presence of a magnesium or rare earth element. The use of especially magnesium chloride is problematic as chloride corrodes steel and, consequently, destroys the reactors used for the reactions.

The preparation of 2-(meth)acryloyloxy benzoic acid esters in the presence of a mixture of an earth alkaline salt and an alkaline salt as catalyst is not described.

JP 2022-147037 relates to a method for reacting 2-hydroxy-4'-(2-hydroxyethoxy)-2-methylpropiophenone with (meth)acrylic anhydride in the presence of a lithium, magnesium or calcium salt.

The preparation of 2-(meth)acryloyloxy benzoic acid esters in the presence of a mixture of an earth alkaline salt and an alkaline salt as catalyst is not described.

JP 2022-147036 relates to a method for producing benzophenone methacrylate with (meth)acrylic anhydride in the presence of a lithium, magnesium or calcium salt.

The preparation of 2-(meth)acryloyloxy benzoic acid esters in the presence of a mixture of an earth alkaline salt and an alkaline salt as catalyst is not described.

It was an object of the present invention to provide a solvent-free method for preparing 2-(meth)acryloyloxy benzoic acid esters in high yield and purity which can be used in large scales and avoids the preparation of equimolar amounts of solid by-products which have to be removed by intensive work-up to isolate the desired product.

It was surprisingly found that the sterically hindered alcohols 2-hydroxybenzoates can be converted to the respective 2-(meth)acryloyloxy benzoic acid esters using (meth)acrylic anhydride as the reagent in the presence of a mixture of an earth alkaline salt and an alkaline salt as catalyst.

Due to the steric hinderance of the alcohol, the commonly known equimolar reactions using known catalysts as described above are not feasible.

### Detailed description of the invention

The present invention is directed to a process for preparing 2-(meth)acryloyloxy benzoic acid esters of general formula (I) 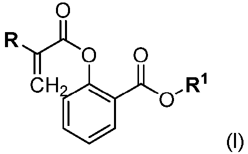 wherein
R denotes H or CH₃ and
**R¹** denotes a C5-7 cycloalkyl group which is unsubstituted or substituted with one, two or three substituents selected from the group consisting of straight-chained or branched C1-6 alkyl, straight-chained or branched C1-6 alkyloxy, -O(CO)-(C1-6)alkyl, -COO-(C1-6)alkyl, or
**R¹** denotes a residue of general formula (II) 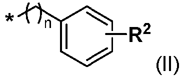
wherein
**R²** denotes a straight-chained or branched C1-6 alkyl group or a straight-chained or branched C1-6 alkyloxy group and
n denotes an integer 0, 1 or 2,
the process comprising the step of:
(a) reacting a 2-hydroxybenzoate of general formula (III): 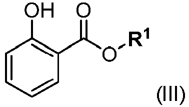 wherein
   **R¹** is defined as disclosed herein before,
   with (meth)acrylic acid anhydride of general formula (IV) 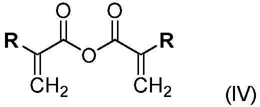
   wherein
   R denotes H or CH₃,
   in the presence of a mixture of a catalyst 1 and a catalyst 2 and at least one stabilizer, characterized in that the reaction takes place in the absence of any solvent.

A further object of the present invention is directed to a process for preparing 2-(meth)acryloyloxy benzoic acid esters of general formula (I), wherein R denotes H or CH₃ and **R¹** denotes a cyclohexyl group which is unsubstituted or substituted with one, two or three substituents selected from the group consisting of straight-chained or branched C1-3 alkyl and straight-chained or branched C1-3 alkyloxy, or a benzyl group.

A further embodiment is directed to the process as outlined further above, wherein the 2-(meth)acryloyloxy benzoic acid ester is 3,3,5-trimethylcyclohexyl salicyl (meth)acrylate of general formula (la): 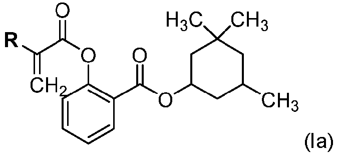 wherein R denotes H or CH₃,
the process comprising the step of:
(a) reacting 3,3,5-trimethylcyclohexyl-2-hydroxybenzoate of formula (Illa): 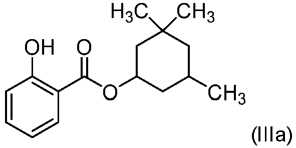
   with (meth)acrylic acid anhydride of general formula (IV) 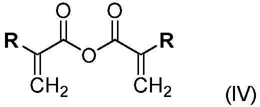
   wherein R denotes H or CH₃,
   in the presence of a mixture of a catalyst 1 and a catalyst 2 and at least one stabilizer, characterized in that the reaction takes place in the absence of any solvent.

The term "(meth)acrylate" refers to both, esters of acrylic acid and esters of methacrylic acid.

The term "(meth)acrylic acid anhydride" refers to both, acrylic acid anhydride and methacrylic acid anhydride.

The term "(meth)acrylic acid" refers to both, acrylic acid and methacrylic acid.

The C5-7 cycloalkyl group for use in accordance with the present invention can be selected from the group consisting of cyclopentyl, cyclohexyl and cycloheptyl.

The straight-chained or branched C1-6 alkyl group for use in accordance with the present invention can be selected from the group consisting of methyl, ethyl, propyl, isopropyl, butyl, isobutyl, tert-butyl, pentyl and hexyl.

The straight-chained or branched C1-3 alkyl group for use in accordance with the present invention can be selected from the group consisting of methyl, ethyl, propyl and isopropyl.

The straight-chained or branched C1-6 alkyloxy group for use in accordance with the present invention can be selected from the group consisting of methyloxy, ethyloxy, propyloxy, isopropyloxy, butyloxy, isobutyloxy, tert-butyloxy, pentyloxy and hexyloxy.

The straight-chained or branched C1-3 alkyloxy group for use in accordance with the present invention can be selected from the group consisting of methyloxy, ethyloxy, propyloxy and isopropyloxy.

In accordance with the present invention, suitable catalysts 1 are selected from the group consisting of earth alkaline salts and the catalysts 2 is selected from the group consisting of alkaline salts.

In a preferred embodiment, the catalyst 1 is selected from the group consisting of calcium oxide and calcium hydroxide and the catalyst 2 is selected from the group consisting of lithium chloride and lithium hydroxide.

In accordance with the present invention, the catalyst 1 and the catalyst 2 are both used in equal amounts of 1 mol% to 3 mol%, preferably 2 mol%, based on the amount of the 2-hydroxybenzoate of general formula (III) or (IIIa) used in the reaction.

Suitable stabilizers are known to those skilled in the art. They include, for example, phenothiazine, substances having an oxyl radical, such as 2,2,6,6-tetramethylpiperidinyl-N-oxyl (TEMPO), 4-hydroxy-2,2,6,6-tetramethylpiperidinyl-N-oxyl (TEMPOL) or 4-(meth)acryloyloxy-2,2,6,6-tetramethylpiperidinyl-N-oxyl (TEMPOL-(meth)acrylate) and also phenol derivatives such as hydroquinone monomethyl ether (HQME), 2,4-dimethyl-6-tert-butylphenol (DMBP), 2,6-di-tert-butylphenol or 4-methyl-2,6-di-tert-butylphenol (BHT). Mixtures of different stabilizers can also be used. Preference is given to using phenothiazine, HQME, DMBP, BHT and mixtures of these substances.

The applications of 2-(meth)acryloyloxy benzoic acid esters (I) and especially 3,3,5-trimethylcyclohexyl salicyl (meth)acrylate (Ia) generally require colourless products. Therefore, for these unsaturated compounds, preference is given to using non-colouring stabilizers or colouring stabilizers in very small amounts. The used amount of stabilizer is dependent on the starting materials.

The amount of stabilizer at the start of the reaction is adjusted to be between 1 ppm and 5000 ppm based on the amount of the 2-hydroxybenzoate of general formula (III) or (Illa) used as starting material. Preferably, the amount of stabilizer at the start of the reaction is adjusted to be between 1 ppm and 3000 ppm, particularly preferably between 1 ppm and 2000 ppm, based on the amount of the 2-hydroxybenzoate of general formula (III) or (Illa) used as starting material.

The reaction is carried out in a suitable reaction vessel that is equipped with a stirrer and a temperature control system under air conditions.

The reaction may be carried out at standard pressure or elevated pressure.

The reaction temperature is generally in the range of 95°C to 115°C, preferably 100°C to 110°C.

The reaction is usually run until all starting material is reacted (GC control).

In accordance with the present invention, reaction times of 4 hours up to 15 hours are preferred.

In order to completely transfer the sterically hindered alcohol to the corresponding product, the use of an excess of (meth)acrylic acid anhydride is necessary.

In accordance with the present invention, 1.5 to 2.5 mol equivalents, preferably about 2 mol equivalents of (meth)acrylic acid anhydride are used, based on the amount of the 2-hydroxybenzoate of general formula (III) or (Illa) used as starting material.

A further object of the present invention is directed to the process comprising step (a) as outlined further above and comprising one or more further steps selected from the group consisting of filtration to remove the heterogenous catalyst, aqueous work-up, distillation to remove low boiling by-products and further distillation to separate the crude product from high-boiling by-products.

To remove the catalyst from the product retrieved in step (a) as outlined further above, the process mixture retrieved in step (a) can be either filtered or diluted with water to dissolve the catalyst in the aqueous phase. To separate the aqueous phase of the mixture, a phase separation can be carried out. This phase separation can be done by commonly known methods, for example by using simple extraction or methods based on gravity like centrifuge, separator or coalescer. In accordance with the present invention, the preferred separation method is by using a centrifuge.

A further object of the present invention is therefore directed to the process comprising step (a) as outlined further above and further comprising the following step (b):
(b) filtering the process mixture retrieved in step (a) or mixing the process mixture retrieved in step (a) with water followed by phase separation to remove the catalyst.

The present invention is intended to be described in more detail below using the examples and comparative examples, without this giving rise to any restriction.

### Experimental Part

### Abbreviations

- CE: comparative example
- homosalate: 3,3,5-trimethylcyclohexyl-2-hydroxybenzoate
- HSMA: homosalate methacrylate = 3,3,5-trimethylcyclohexyl salicyl (meth)acrylate
- MA: methacrylic acid
- MAAH: methacrylic acid anhydride

### Analytical Measurements

### Determination of the process mixture composition by gas chromatography (GC):

To determine the process mixture composition, a sample of the composition in acetone (20% solution) was investigated by gas chromatographic analysis. For this purpose, an "Agilent 7820" instrument from Agilent was used with a column of the DB5 type (length: 30.0 m, diameter: 250.00 µm, film thickness: 0.25 µm). The injection amount was 0.2 µL (split 1:60). The instrument was operated at an injector temperature of 300°C and a detector temperature of 300°C. The following temperature program was used: 80°C for 2 minutes, heating to 300°C at a heating rate of 16°C per minute and maintaining the final temperature for further 6 minutes.

### Examples

### Preparation of a stock solution for the preparation of examples 1-8 and comparative examples 1-13 targeted molar ratio of homosalate : methacrylic acid anhydride = 1:2

78.7 g of homosalate (0.3 mol) and 92.5 g of methacrylic acid anhydride (0.6 mol) were combined with 17.2 mg of phenothiazine (100 ppm) and stirred to obtain a stock solution with a molar ratio of homosalate : methacrylic acid anhydride of 1:2.

### General procedure for the preparation of examples 1-8 and comparative examples 1-13

7.0 g samples of the corresponding stock solution were placed in a 15 mL pressure tube with a Teflon^{®} plug and a magnetic agitator. To this solution, the catalyst was added as indicated in Table 1 (mol% with respect to the amount of homosalate). The pressure tube was tightly closed. Subsequently, the pressure tube was placed in an aluminum heating block with an integrated magnetic stirrer and stirred. The respective time and temperature can be found in Table 1. Samples for GC analysis were filtered through a syringe filter unit and diluted with acetone (20% solution) prior to analysis.

**Table 1: Reaction conditions**

| Example | Catalyst | Catalyst amount | Catalyst amount | Reaction time | Reaction temperature |
|---|---|---|---|---|---|
| # | | [g] | [mol%] | [h] | [°C] |
| 1 | CaO/ LiOH | 0.0134/ 0.0057 | 2/2 | 4 | 100 |
| 2 | CaO/ LiOH | 0.0134/ 0.0057 | 2/2 | 8 | 100 |
| 3 | CaO/ LiCl | 0.0134/ 0.0102 | 2/2 | 4 | 100 |
| 4 | CaO/ LiCl | 0.0134/ 0.0102 | 2/2 | 8 | 100 |
| 5 | Ca(OH)₂/ LiOH | 0.0178/ 0.0057 | 2/2 | 4 | 100 |
| 6 | Ca(OH)₂/ LiOH | 0.0178/ 0.0057 | 2/2 | 8 | 100 |
| 7 | Ca(OH)₂/ LiCl | 0.0178/ 0.0102 | 2/2 | 4 | 100 |
| 8 | Ca(OH)₂/ LiCl | 0.0178/ 0.0102 | 2/2 | 8 | 100 |
| CE 1 | CaO | 0.0268 | 4 | 4 | 100 |
| CE 2 | CaO | 0.0268 | 4 | 8 | 100 |
| CE 3 | Ca(OH)₂ | 0.0364 | 4 | 4 | 90 |
| CE 4 | Ca(OH)₂ | 0.0364 | 4 | 8 | 90 |
| CE 5 | Ca(OH)₂ | 0.0364 | 4 | 4 | 100 |
| CE 6 | LiOH | 0.0118 | 4 | 4 | 90 |
| CE 7 | LiOH | 0.0118 | 4 | 8 | 90 |
| CE 8 | LiOH | 0.0118 | 4 | 4 | 100 |
| CE 9 | LiOH | 0.0118 | 4 | 8 | 100 |
| CE 10 | LiCl | 0.0209 | 4 | 4 | 90 |
| CE 11 | LiCl | 0.0209 | 4 | 8 | 90 |
| CE 12 | LiCl | 0.0209 | 4 | 4 | 100 |
| CE 13 | LiCl | 0.0209 | 4 | 8 | 100 |

| | | | | | |
|---|---|---|---|---|---|
| CE = comparative example | | | | | |

Examples 1-8 are in accordance with the present invention and disclose the preparation of 3,3,5-trimethylcyclohexyl salicyl (meth)acrylate from 3,3,5-trimethylcyclohexyl-2-hydroxybenzoate by using a mixture of an earth alkaline salt and an alkaline salt as catalyst, namely the combinations CaO/LiOH, CaO/LiCl, Ca(OH)₂/LiOH or Ca(OH)₂/LiCl.

Comparative examples 1-13 use either an earth alkaline salt or an alkaline salt as single catalyst for the same reaction.

The following Table 2 shows the yield of HSMA, residual amount of homosalate as well as the amount of high boiling impurities of the examples and comparative examples. All amounts were determined by gas chromatography and are reported in area %.

**Table 2: Residual homosalate, homosalate methacrylate (HSMA) conversion and high boiler impurities determined by gas chromatography (in area %)**

| Example | Catalyst | Time | Temperature | Homosalate | HSMA | High boilers | HSMA : Homo- |
|---|---|---|---|---|---|---|---|
| # | [mol%] | [h] | [°C] | [area %] | [area %] | [area %] | salate |
| 1 | CaO/ LiOH (2/2) | 4 | 100 | 2.7 | 60.7 | 1.8 | 22.6 |
| 2 | CaO/ LiOH (2/2) | 8 | 100 | 1.1 | 62.6 | 2.2 | 56.0 |
| 3 | CaO/LiCl (2/2) | 4 | 100 | 2.0 | 61.4 | 1.6 | 31.4 |
| 4 | CaO/LiCl (2/2) | 8 | 100 | 1.0 | 62.7 | 2.1 | 60.8 |
| 5 | Ca(OH)₂/ LiOH (2/2) | 4 | 100 | 2.2 | 60.9 | 1.7 | 27.6 |
| 6 | Ca(OH)₂/ LiOH (2/2) | 8 | 100 | 1.3 | 62.1 | 2.2 | 46.9 |
| 7 | Ca(OH)₂/ LiCl (2/2) | 4 | 100 | 2.1 | 61.2 | 1.7 | 29.5 |
| 8 | Ca(OH)₂/ LiCl (2/2) | 8 | 100 | 1.1 | 62.6 | 2.1 | 56.5 |
| CE 1 | CaO (4) | 4 | 100 | 3.7 | 59.7 | 1.2 | 16.0 |
| CE 2 | CaO (4) | 8 | 100 | 3.6 | 60.0 | 1.2 | 16.6 |
| CE 3 | Ca(OH)₂ (4) | 4 | 90 | 18.5 | 43.5 | 0.4 | 2.35 |
| CE 4 | Ca(OH)₂ (4) | 8 | 90 | 5.0 | 59.7 | 1.1 | 12.0 |
| CE 5 | Ca(OH)₂ (4) | 4 | 100 | 6.6 | 57.5 | 1.1 | 8.7 |
| CE 6 | LiOH (4) | 4 | 90 | 18.5 | 50.6 | 1.7 | 2.7 |
| CE 7 | LiOH (4) | 8 | 90 | 12.3 | 57.8 | 2.6 | 4.7 |
| CE 8 | LiOH (4) | 4 | 100 | 13.6 | 47.5 | 1.9 | 3.5 |
| CE 9 | LiOH (4) | 8 | 100 | 5.1 | 57.1 | 3.0 | 11.2 |
| CE 10 | LiCl (4) | 4 | 90 | 36.7 | 29.3 | 0.6 | 0.8 |
| CE 11 | LiCl (4) | 8 | 90 | 29.6 | 37.6 | 1.2 | 1.3 |
| CE 12 | LiCl (4) | 4 | 100 | 27.1 | 31.1 | 0.6 | 1.1 |
| CE 13 | LiCl (4) | 8 | 100 | 20.4 | 39.2 | 1.8 | 1.9 |

### Conclusions:

Table 2 shows that the use of a catalyst mixtures in accordance with the invention leads to the desired product in good yields, with equal to or less than 3% of residual starting material and low amounts of high boiling by-products.

The use of only a single catalyst usually leads to the desired product which still contains more than 3% of the starting material. In addition, when using an alkaline salt as single catalyst, the yield is low.

## Claims

1. Process for preparing 2-(meth)acryloyloxy benzoic acid esters of general formula (I) 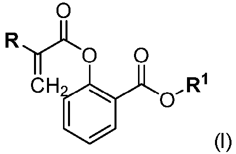 wherein
R denotes H or CH₃ and
**R¹** denotes a C5-7 cycloalkyl group which is unsubstituted or substituted with one, two or three substituents selected from the group consisting of straight-chained or branched C1-6 alkyl, straight-chained or branched C1-6 alkyloxy, -O(CO)-(C1-6)alkyl, -COO-(C1-6)alkyl, or
**R¹** denotes a residue of general formula (II) 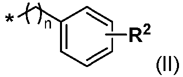
wherein
**R²** denotes a straight-chained or branched C1-6 alkyl group or a straight-chained or branched C1-6 alkyloxy group and
n denotes an integer 0, 1 or 2,
the process comprising the step of:
(a) reacting a 2-hydroxybenzoate of general formula (III): 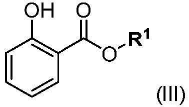 wherein
**R¹** is defined as disclosed herein before,
with (meth)acrylic acid anhydride of general formula (IV) 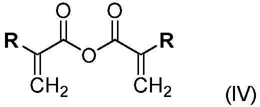
wherein R denotes H or CH₃,
in the presence of a mixture of a catalyst 1 and a catalyst 2 and at least one stabilizer,
**characterized in that** the reaction takes place in the absence of any solvent.

2. The process according to claim 1, wherein **R¹** denotes a cyclohexyl group which is unsubstituted or substituted with one, two or three substituents selected from the group consisting of straight-chained or branched C1-3 alkyl and straight-chained or branched C1-3 alkyloxy, or a benzyl group.

3. The process according to Claim 1 or 2, wherein the catalyst **1** is selected from the group consisting of earth alkaline salts and the catalyst 2 is selected from the group consisting of alkaline salts.

4. The process according to Claim 1, 2 or 3, wherein the catalyst **1** is selected from the group consisting of calcium oxide and calcium hydroxide, and the catalyst 2 is selected from the group consisting of lithium chloride and lithium hydroxide.

5. The process according to Claim 1, 2, 3 or 4, wherein the catalyst 1 and the catalyst 2 are both used in equal amounts of 1 mol% to 3 mol%, preferably 2 mol%, based on the amount of the 2-hydroxybenzoate of general formula (III) used in the reaction.

6. The process according to Claim 1, 2, 3, 4 or 5, wherein the stabilizer is selected from the group consisting of phenothiazine, 2,2,6,6-tetramethylpiperidinyl-N-oxyl (TEMPO), 4-hydroxy-2,2,6,6-tetramethylpiperidinyl-N-oxyl (TEMPOL), 4-(meth)acryloyloxy-2,2,6,6-tetramethylpiperidinyl-N-oxyl (TEMPOL-(meth)acrylate), hydroquinone monomethyl ether (HQME), 2,4-dimethyl-6-tert-butylphenol (DMBP), 2,6-di-tert-butylphenol, 4-methyl-2,6-di-tert-butylphenol (BHT) and mixtures thereof.

7. The process according to Claim 1, 2, 3, 4 or 5, wherein the stabilizer is selected from the group consisting of phenothiazine, hydroquinone monomethyl ether (HQME), 2,4-dimethyl-6-tert-butylphenol (DMBP), 4-methyl-2,6-di-tert-butylphenol (BHT) and mixtures of these substances.

8. The process according to Claim 1, 2, 3, 4, 5, 6 or 7, wherein the stabilizer is used in amounts of 1 ppm to 5000 ppm, preferably 1 ppm to 3000 ppm more preferably 1 ppm to 2000 ppm, based on the amount of the 2-hydroxybenzoate of general formula (III) used as starting material.

9. The process according to Claim 1, 2, 3, 4, 5, 6, 7, or 8, comprising one or more further steps selected from the group consisting of filtration to remove the heterogenous catalyst, aqueous work-up, distillation to remove low boiling by-products and further distillation to separate the crude product from high-boiling by-products.

10. The process according to Claim 1, 2, 3, 4, 5, 6, 7, 8 or 9, wherein (meth)acrylic acid anhydride of general formula (IV) is used in an excess of 1.5 to 2.5 mol equivalents, preferably about 2 mol equivalents, based on the amount of the 2-hydroxybenzoate of general formula (III) used in step (a).

11. The process according to Claim 1, 2, 3, 4, 5, 6, 7, 8, 9 and 10, wherein the 2-(meth)acryloyloxy benzoic acid ester is 3,3,5-trimethylcyclohexyl salicyl (meth)acrylate of general formula (la): 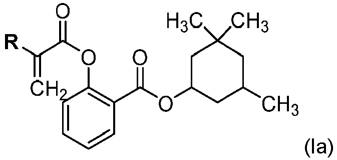 wherein R denotes H or CH₃,
comprising the step of:
(a) reacting 3,3,5-trimethylcyclohexyl-2-hydroxybenzoate of formula (Illa): 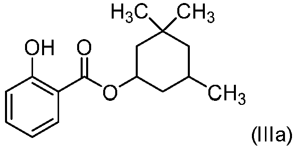
with (meth)acrylic acid anhydride of general formula (IV) 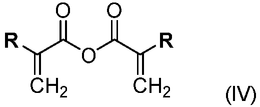
wherein R denotes H or CH₃,
in the presence of a mixture of a catalyst 1 and a catalyst 2 and at least one stabilizer, **characterized in that** the reaction takes place in the absence of any solvent.

12. The process according to Claim 11, wherein the catalyst 1 is selected from the group consisting of calcium oxide and calcium hydroxide, and the catalyst 2 is selected from the group consisting of lithium chloride and lithium hydroxide.

13. The process according to Claim 11 or 12, wherein the catalyst 1 and the catalyst 2 are both used in equal amounts of 1 mol% to 3 mol%, preferably 2 mol%, based on the amount of the 2-hydroxybenzoate of general formula (III) used in the reaction.

14. The process according to Claim 11, 12 or 13, wherein the stabilizer is selected from the group consisting of phenothiazine, hydroquinone monomethyl ether (HQME), 2,4-dimethyl-6-tert-butylphenol (DMBP), 4-methyl-2,6-di-tert-butylphenol (BHT) and mixtures of these substances.

## Patentansprüche

1. Verfahren zur Herstellung von 2-(Meth)acryloyloxy-benzoesäureestern der allgemeinen Formel (I): 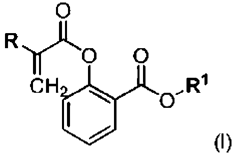 wobei
R H oder CH₃ bedeutet, und
R¹ eine C5-7-Cycloalkylgruppe bedeutet, die unsubstituiert oder mit einem, zwei oder drei Substituenten substituiert ist, die ausgewählt sind aus der Gruppe bestehend aus geradkettigem oder verzweigtem C1-6-Alkyl, geradkettigem oder verzweigtem C1-6-Alkyloxy, -O(CO)-(C1-6)-Alkyl, -COO-(C1-6)-Alkyl,
oder
R¹ einen Rest der allgemeinen Formel (II) bedeutet: 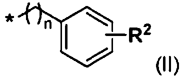
wobei
R² eine geradkettige oder verzweigte C1-6-Alkylgruppe oder eine geradkettige oder verzweigte C1-6-Alkyloxygruppe bedeutet, und
n eine ganze Zahl 0, 1 oder 2 bedeutet,
wobei das Verfahren den folgenden Schritt umfasst:
(a) Umsetzen eines 2-Hydroxybenzoats der allgemeinen Formel (III): 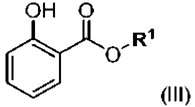
wobei
R¹ wie hierin zuvor offenbart definiert ist,
mit (Meth)acrylsäureanhydrid der allgemeinen Formel (IV): 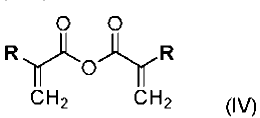
wobei R H oder CH₃ bedeutet,
in Gegenwart einer Mischung aus einem Katalysator 1 und einem Katalysator 2 und mindestens einem Stabilisator, **dadurch gekennzeichnet, dass** die Umsetzung in Abwesenheit von jeglichem Lösungsmittel erfolgt.

2. Verfahren nach Anspruch 1, wobei R¹ eine Cyclohexylgruppe, die unsubstituiert oder mit einem, zwei oder drei Substituenten substituiert ist, ausgewählt aus der Gruppe bestehend aus geradkettigem oder verzweigtem C1-3-Alkyl und geradkettigem oder verzweigtem C1-3-Alkyloxy, oder eine Benzylgruppe bedeutet.

3. Verfahren nach Anspruch 1 oder 2, wobei der Katalysator 1 ausgewählt ist aus der Gruppe bestehend aus Erdalkalisalzen, und der Katalysator 2 ausgewählt ist aus der Gruppe bestehend aus Alkalisalzen.

4. Verfahren nach Anspruch 1, 2 oder 3, wobei der Katalysator 1 ausgewählt ist aus der Gruppe bestehend aus Calciumoxid und Calciumhydroxid, und der Katalysator 2 ausgewählt ist aus der Gruppe bestehend aus Lithiumchlorid und Lithiumhydroxid.

5. Verfahren nach Anspruch 1, 2, 3 oder 4, wobei der Katalysator 1 und der Katalysator 2 beide in gleichen Mengen von 1 Mol-% bis 3 Mol-%, vorzugsweise 2 Mol-% verwendet werden, bezogen auf die bei der Umsetzung verwendete Menge des 2-Hydroxybenzoats der allgemeinen Formel (III).

6. Verfahren nach Anspruch 1, 2, 3, 4 oder 5, wobei der Stabilisator ausgewählt ist aus der Gruppe bestehend aus Phenothiazin, 2,2,6,6-Tetramethylpiperidinyl-N-oxyl (TEMPO), 4-Hydroxy-2,2,6,6-tetramethylpiperidinyl-N-oxyl (TEMPOL), 4-(Meth)acryloyloxy-2,2,6,6-tetramethylpiperidinyl-N-oxyl (TEMPOL-(meth)acrylat), Hydrochinonmonomethylether (HOME), 2,4-Dimethyl-6-tert.-butylphenol (DMBP), 2,6-Di-tert.-butylphenol, 4-Methyl-2,6-di-tert.-butylphenol (BHT) und Mischungen davon.

7. Verfahren nach Anspruch 1, 2, 3, 4 oder 5, wobei der Stabilisator ausgewählt ist aus der Gruppe bestehend aus Phenothiazin, Hydrochinonmonomethylether (HOME), 2,4-Dimethyl-6-tert.-butylphenol (DMBP), 4-Methyl-2,6-di-tert.-butylphenol (BHT) und Mischungen dieser Substanzen.

8. Verfahren nach Anspruch 1, 2, 3, 4, 5, 6 oder 7, wobei der Stabilisator in Mengen von 1 ppm bis 5000 ppm, vorzugsweise 1 ppm bis 3000 ppm, bevorzugter 1 ppm bis 2000 ppm verwendet wird, bezogen auf die Menge des als Ausgangsmaterial verwendeten 2-Hydroxybenzoats der allgemeinen Formel (III).

9. Verfahren nach Anspruch 1, 2, 3, 4, 5, 6, 7 oder 8, umfassend einen oder mehrere weitere Schritte ausgewählt aus der Gruppe bestehend aus Filtration zur Entfernung des heterogenen Katalysators, wässriger Aufarbeitung, Destillation zur Entfernung niedrigsiedender Nebenprodukte und weiterer Destillation zur Abtrennung des Rohprodukts von hochsiedenden Nebenprodukten.

10. Verfahren nach Anspruch 1, 2, 3, 4, 5, 6, 7, 8 oder 9, wobei (Meth)acrylsäureanhydrid der allgemeinen Formel (IV) in einem Überschuss von 1,5 bis 2,5 Moläquivalenten, vorzugsweise etwa 2 Moläquivalenten verwendet wird, bezogen auf die Menge des in Schritt (a) verwendeten 2-Hydroxybenzoats der allgemeinen Formel (III).

11. Verfahren nach Anspruch 1, 2, 3, 4, 5, 6, 7, 8, 9 und 10, wobei der 2-(Meth)acryloyloxybenzoesäureester 3,3,5-Trimethylcyclohexylsalicyl(meth)acrylat der allgemeinen Formel (Ia) ist: 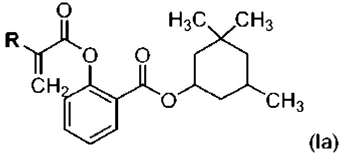
wobei R H oder CH₃ bedeutet,
welches den folgenden Schritt umfasst:
(a) Umsetzen von 3,3,5-Trimethylcyclohexyl-2-hydroxybenzoat der Formel (IIIa): 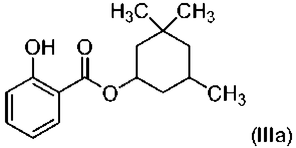
mit (Meth)acrylsäureanhydrid der allgemeinen Formel (IV): 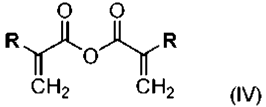
wobei R H oder CH₃ bedeutet,
in Gegenwart einer Mischung aus einem Katalysator 1 und einem Katalysator 2 und mindestens einem Stabilisator, **dadurch gekennzeichnet, dass** die Umsetzung in Abwesenheit von jeglichem Lösungsmittel erfolgt.

12. Verfahren nach Anspruch 11, wobei der Katalysator 1 ausgewählt ist aus der Gruppe bestehend aus Calciumoxid und Calciumhydroxid, und der Katalysator 2 ausgewählt ist aus der Gruppe bestehend aus Lithiumchlorid und Lithiumhydroxid.

13. Verfahren nach Anspruch 11 oder 12, wobei der Katalysator 1 und der Katalysator 2 beide in gleichen Mengen von 1 Mol-% bis 3 Mol-%, vorzugsweise 2 Mol-% verwendet werden, bezogen auf die bei der Umsetzung verwendete Menge des 2-Hydroxybenzoats der allgemeinen Formel (III).

14. Verfahren nach Anspruch 11, 12 oder 13, wobei der Stabilisator ausgewählt ist aus der Gruppe bestehend aus Phenothiazin, Hydrochinonmonomethylether (HOME), 2,4-Dimethyl-6-tert.-butylphenol (DMBP), 4-Methyl-2,6-di-tert.-butylphenol (BHT) und Mischungen dieser Substanzen.

## Revendications

1. Procédé de préparation d'esters d'acide 2-(méth)acryloyloxybenzoïque de la formule générale (I) 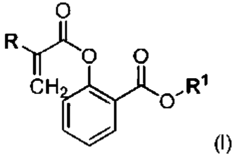 dans laquelle
R désigne H ou CH₃ et
R¹ désigne un groupe cycloalkyle en C5-7 qui est non substitué ou substitué par un, deux ou trois substituants choisis dans un groupe constitué d'un alkyle en C1-6 à chaîne droite ou ramifié, d'un alkoxyle en C1-6 à chaîne droite ou ramifié, d'un -O(CO)-(C1-6)alkyle, d'un -COO-(C1-6)alkyle,
ou
R¹ désigne un radical de la formule générale (II) 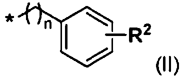
dans laquelle
R² désigne un groupe alkyle en C1-6 à chaîne droite ou ramifié ou un groupe alkoxyle en C1-6 à chaîne droite ou ramifié et
n désigne un entier 0, 1 ou 2,
le procédé comprenant les étapes de :
(a) mise en réaction d'un 2-hydroxybenzoate de la formule générale (III) : 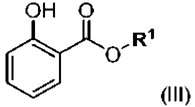 dans laquelle
R¹ est défini comme divulgué ici précédemment,
avec un anhydride d'acide (méth)acrylique de la formule générale (IV) 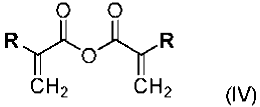
dans laquelle R désigne H ou CH₃,
en présence d'un mélange d'un catalyseur 1 et d'un catalyseur 2 et d'au moins un stabilisant,
**caractérisé en ce que** la réaction a lieu en l'absence de tout solvant.

2. Procédé selon la revendication 1, dans lequel R¹ désigne un groupe cyclohexyle qui est non substitué ou substitué par un, deux ou trois substituants choisis dans un groupe constitué d'un groupe alkyle en C1-3 linéaire ou ramifié et d'un groupe alkoxyle en C1-3 linéaire ou ramifié, ou d'un groupe benzyle.

3. Procédé selon la revendication 1 ou 2, dans lequel le catalyseur 1 est choisi dans un groupe constitué des sels alcalino-terreux et le catalyseur 2 est choisi dans un groupe constitué des sels alcalins.

4. Procédé selon la revendication 1, 2 ou 3, dans lequel le catalyseur 1 est choisi dans un groupe constitué de l'oxyde de calcium et de l'hydroxyde de calcium, et le catalyseur 2 est choisi dans un groupe constitué du chlorure de lithium et de l'hydroxyde de lithium.

5. Procédé selon la revendication 1, 2, 3 ou 4, dans lequel le catalyseur 1 et le catalyseur 2 sont tous les deux utilisés en quantités égales de 1 % en moles à 3 % en moles, de préférence de 2 % en moles, par rapport à la quantité du 2-hydroxybenzoate de la formule générale (III) utilisée dans la réaction.

6. Procédé selon la revendication 1, 2, 3, 4 ou 5, dans lequel le stabilisant est choisi dans un groupe constitué du phénothiazine, du 2,2,6,6-tétraméthylpipéridinyl-N-oxyl (TEMPO), du 4-hydroxy-2,2,6,6-tétraméthylpipéridinyl-N-oxyl (TEMPOL), du 4-(méth)acryloyloxy-2,2,6,6-tétraméthylpipéridinyl-N-oxyl (TEMPOL-(méth)acrylate), de l'éther monométhylique d'hydroquinone (HQME), du 2,4-diméthyl-6-tert-butylphénol (DMBP), du 2,6-di-tert-butylphénol, du 4-méthyl-2,6-di-tert-butylphénol (BHT) et de leurs mélanges.

7. Procédé selon la revendication 1, 2, 3, 4 ou 5, dans lequel le stabilisant est choisi dans un groupe constitué de la phénothiazine, de l'éther monométhylique d'hydroquinone (HQME), du 2,4-diméthyl-6-tert-butylphénol (DMBP), du 4-méthyl-2,6-di-tert-butylphénol (BHT) et des mélanges de ces substances.

8. Procédé selon la revendication 1, 2, 3, 4, 5, 6 ou 7, dans laquelle le stabilisant est utilisé en quantités de 1 ppm à 5 000 ppm, de préférence de 1 ppm à 3 000 ppm, plus préférablement de 1 ppm à 2 000 ppm, par rapport à la quantité de 2-hydroxybenzoate de la formule générale (III) utilisée comme matériau de départ.

9. Procédé selon la revendication 1, 2, 3, 4, 5, 6, 7 ou 8, comprenant une ou plusieurs étapes supplémentaires sélectionnées dans un groupe constitué d'une filtration pour éliminer le catalyseur hétérogène, d'un traitement aqueux, d'une distillation pour éliminer les sous-produits à bas point d'ébullition et d'une distillation supplémentaire pour séparer le produit brut des sous-produits à haut point d'ébullition.

10. Procédé selon la revendication 1, 2, 3, 4, 5, 6, 7, 8 ou 9, dans lequel l'anhydride d'acide (méth)acrylique de la formule générale (IV) est utilisé en un excès de 1,5 à 2,5 équivalents molaires, de préférence environ 2 équivalents molaires, par rapport à la quantité de 2-hydroxybenzoate de la formule générale (III) utilisée dans l'étape (a).

11. Procédé selon la revendication 1, 2, 3, 4, 5, 6, 7, 8, 9 et 10, dans lequel l'ester d'acide 2-(méth)acryloyloxy benzoïque est le (méth)acrylate de 3,3,5-triméthylcyclohexylsalicyle de la formule générale (Ia) : 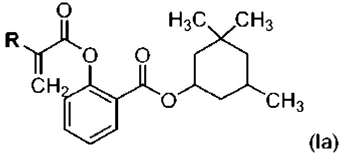
dans laquelle R désigne H ou CH₃,
comprenant l'étape de :
(a) mise en réaction du 3,3,5-triméthylcyclohexyle-2-hydroxybenzoate de la formule (IIIa) : 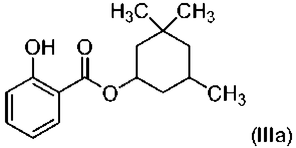
avec un anhydride d'acide (méth)acrylique de la formule générale (IV) 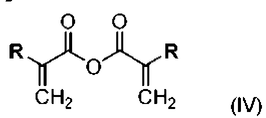
dans laquelle R désigne H ou CH₃,
en présence d'un mélange d'un catalyseur 1 et d'un catalyseur 2 et d'au moins un stabilisant,
**caractérisé en ce que** la réaction a lieu en l'absence de tout solvant.

12. Procédé selon la revendication 11, dans lequel le catalyseur 1 est choisi dans un groupe constitué de l'oxyde de calcium et de l'hydroxyde de calcium, et le catalyseur 2 est choisi dans un groupe constitué du chlorure de lithium et de l'hydroxyde de lithium.

13. Procédé selon la revendication 11 ou 12, dans lequel le catalyseur 1 et le catalyseur 2 sont tous les deux utilisés en quantités égales de 1 % en moles à 3 % en moles, de préférence de 2 % en moles, par rapport à la quantité de 2-hydroxybenzoate de la formule générale (III) utilisée dans la réaction.

14. Procédé selon la revendication 11, 12 ou 13, dans lequel le stabilisant est choisi dans un groupe constitué de la phénothiazine, de l'éther monométhylique d'hydroquinone (HQME), du 2,4-diméthyl-6-tert-butylphénol (DMBP), du 4-méthyl-2,6-di-tert-butylphénol (BHT) et des mélanges de ces substances.
